(19) Europäisches Patentamt
European Patent Office
Office européen des brevets

(11) Numéro de publication: **0 003 190**
**B1**

(12) **FASCICULE DE BREVET EUROPEEN**

(21) Numéro de dépôt: **79400004.2**

(22) Date de dépôt: **04.01.79**

(51) Int. Cl.³: **C 07 D 207/40,**
**C 10 M 1/32**

# (54) Nouvelles compositions à base d'alcénylsuccinimides dérivés de la tris(amino-5 thia-3 pentyl)amine, leur procédé de préparation et leur application comme additifs pour lubrifiants

(30) Priorité: **11.01.78 FR 7800625**

(43) Date de publication de la demande:
**25.07.79 Bulletin 79/15**

(45) Mention de la délivrance du brevet:
**23.07.80 Bulletin 80/15**

(84) Etats contractants désignés:
**BE CH DE GB NL SE**

(56) Documents cités:
**FR - A - 1 460 163**
**US - A - 3 865 813**

(73) Titulaire: **Societe Orogil**
**25, quai Paul Doumer**
**F - 92408 Courbevoie (FR)**

(72) Inventeur: **Soula, Gérard**
**33, rue Nungesser**
**F - 69330 Meyzieu (FR)**

(74) Mandataire: **Fabre, Madeleine-France**
**Rhone Poulenc Service Brevets Chimie et Polymères B.P. 753**
**F - 75360 Paris Cedex 08 (FR)**

EP 0 003 190 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouvelles compositions à base d'alcénylsuccinimides dérivés de la tris(amino-5 thia-3-pentyl)amine, leur procédé de préparation et leur application comme additifs pour lubrifiants

La présente invention a pour objet de nouvelles compositions à base d'alcénylsuccinimides dérivés de la tris(amino-5 thia-3 pentyl)amine, leur procédé de préparation et leur application comme additifs pour huiles lubrifiantes.

Il est déjà connu de faire réagir les anhydrides alcénylsucciniques sur des monoamines aliphatiques, des amines aromatiques, hétérocycliques etc...., des alcoylidénepolyamines, des polyoxyalcoylidèneamines etc.... et d'utiliser les alcénylsuccinimides ainsi obtenus comme additifs pour huiles lubrifiantes.

La demanderesse a trouvé des compositions à base de nouveaux alcénylsuccinimides conférant aux huiles lubrifiantes des propriétés détergentes-dispersantes et anti-rouilles améliorées.

Les nouvelles compositions objet de la présente invention sont caractérisées en ce qu'elles comprennent au moins un alcénylsuccinimide de formule:

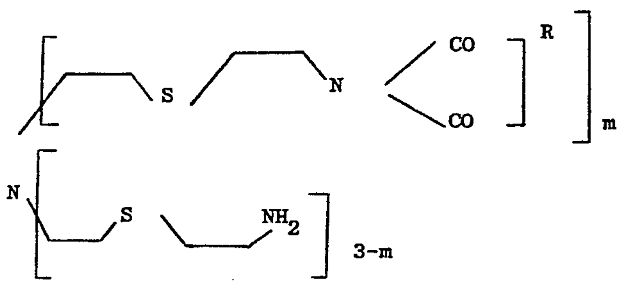

où R représente un groupe alcényl contenant de 20 à 200 atomes de carbone et m est un nombre entier compris entre 1 et 3.

Les nouvelles compositions objet de l'invention peuvent être préparées par action d'un anhydride alcénylsuccinique dans lequel le groupe alcényle renferme de 20 à 200 atomes de carbone, avec de la tris(amino-5 thia-3 pentyl)amine, à une températur comprise entre 120 et 230°C, de préférence entre 140 et 180°C, selon un rapport nombre d'équivalents amine/nombre d'équivalents acides compris entre 0,4 et 0,6.

On entend par nombre d'équivalents amine le nombre de motifs $—NH_2$ de la tris(amino-5 thia-3 pentyl)amine; on entend par nombre d'équivalents acide le nombre de motifs

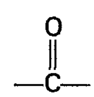

de l'anhydride alcénylsuccinique mis en oeuvre; théoriquement il faut 1 équivalent amine pour 2 équivalents acide pour former un noyau succinimide.

La réaction est éventuellement réalisée en présence d'un diluant pour diminuer la viscosité du milieu réactionnel; ledit diluant sera de préférence choise parmi les huiles lubrifiantes pouvant servir d'huiles de base dans les compositions lubrifiantes, huiles de base dont des exemples seront donnés ci-après.

Les anhydrides alcénylsucciniques mis en oeuvre sont préparés d'une manière connue, par exemple par condensation par voie thermique (brevet américain n° 3 306 907) de l'anhydride maléique sur une polyoléfine des poids moléculaire moyen compris entre 400 et 4000. Ladite polyoléfine est choisie parmi les oligomères ou polymères d'oléfines en $C_2$—$C_{30}$ éventuellement ramifiées, ou les copolymères desdites oléfines entre elles ou avec des comonomères diéniques ou vinylaromatiques; parmi ces polyoléfines ou peut citer de préférence les oligomères d'$\alpha$-monooléfines en $C_2$—$C_{20}$ tels que les oligomères de l'éthylèene, du proplyène, du butène-1, de l'isobutène, du cyclohexyl-3-butène-1, du méthyl-2 propyl-5 hexène-1, les copolymères des ces $\alpha$ oléfines entre elles ou avec des oléfines internes, ainsi que les copolymères de l'isobutène avec un comonomère choisi parmi le butadiène, le styrène, l'hexadiène/1,3 ou les diènes et triènes conjugués ou non.

L'opèration de condensation peut également être réalisée en présence de chlore (brevet amèricain n° 3 231 587 et brevet belge n° 805 486), d'iode (brevet anglais n° 1 356 802) ou de brome (demande de brevet français n° 74 18915 déposée le 31 Mai 1974 au nom de la Société Française dite RHONE-PROGIL); cette opération peut être aussi réalisée au départ de polyoléfines monochlorées ou monobromées, tel que cela est signalé dans le brevet français publiè sous le n° 2 042 558.

La tris(amino-5 thia-3 pentyl)amine mise en oeuvre peut être préparée par action de la tris(éthanethiol)amine sur de l'aziridine selon un rapport molaire aziridine/tris(éthanethiol) amine compris entre 3 et 3,5, de préférence entre 3 et 3,2 à une température comprise entre 30 et 85°C, de préférence entre 45 et 80°C, puis séparation de la tris(amino-5 thia-3 pentyl)amine obtenue.

La réaction entre l'aziridine et la tris(éthanethiol) amine est réalisée en présence d'un solvant tel que le mèthanol, l'éthanol, le chloroforme, le chlorure de méthylène, le chlorobenzéne . . .; celle-ci dure environ de 0,5 à 3 heures.

La présente invention vise également les huiles lubrifiantes améliorées par addition de 1 à 10 % de leurs poids, des compositions ci-dessus décrites apportant leurs propriétés détergentes-dispersantes, antirouille et antimousse auxdites huiles.

Les huiles lubrifiantes que l'on peut utiliser peuvent être choisies parmi des huiles lubrifiantes très variées, comme les huiles lubrifiantes de base naphténique, de base paraffinique, de base mixte, d'autres lubrifiants hydrocarbonés, par exemple des huiles lubrifiantes dérivées de produits de la houille, et des huiles synthétiques, par exemple des polymères d'alkylène, des polymères du type oxyde d'alkylène et leurs dérivés, y compris les polymères d'oxyde d'alkylène préparés en polymérisant l'oxyde d'alkylène en présence d'eau ou d'alcools, par exemple l'alcool éthylique, les esters d'acides dicarboxyliques, les esters liquides d'acides du phosphore, des alkylbenzènes et des dialkyl benzènes, des polyphényles, des alkyl biphényl éthers, des polymères du silicium.

La quantité de nouveaux additifs à ajouter est fonction de l'utilisation future de l'huile lubrifiante à améliorer; ainsi pour huile pour moteur à essence la quantité d'additif à ajouter sera de 1 à 7%; pour une huile pour moteur diesel elle sera de 4 à 10 %.

Les huiles lubrifiantes améliorées peuvent également contenir les adjuvants anti-oxydants, anticorrosion etc. . . .

Les exemples suivants sont donnés à titre indicatif et ne peuvent être considérés comme une limite du domaine et de l'esprit de l'invention.

## Exemple 1

Dans un ballon tricol de 250 cm3 équipé d'un agitateur mécanique, d'un thermomètre et d'une ampoule à brome on introduit 120 g d'anhydride polyisobuténylsuccinique obtenu à partir d'anhydride maléique et de polyisobutène dont le poids moléculaire moyen est de 900. L'indice d'acide de cet anhydride alcénylsuccinique est de 70 mg de KOH/g. Le produit est chauffé à 155°C. On introduit alors en 30 minutes, 8,3 g de tris(amino-5 thia-3 pentyl) amine. Après addition de l'amine, le mélange est placé sous vide (20 mm Hg) à 155°C pendant 2 heures. On obtient un produit limpide composé essentiellement d'un tris succinimide dérivé de l'amine introduite. La composition chimique de ce produit est la suivante:

azote : % mesuré = 1,05   % théorie = 1,10
soufre: % mesuré = 1,92   % théorie = 1,91

La tris(amino-5 thia-3 pentyl)amine mise en oeuvre peut être préparée de la manière suivante:

— *Synthèse de la tris(amino-5 thia-3 pentyl)amine*

Dans un ballon tricol de 500 cm3 composé d'un agitateur mécanique, d'un thermomètre et d'une ampoule à brome, on introduit 19,7 g soit 0,1 mole de tris(thioéthanol)amine en solution dans 250 cm3 de méthanol. Le mélange est chauffé à 45°C. On introduit alors 0,3 mole d'aziridine (15,5 ml) diluée dans 20 cm3 de méthanol, en 30 mn à l'aide de l'ampoule à brome. Le mélange est ensuite porté à la température de reflux du méthanol (64°C) pendant 3 heures et enfin le méthanol est évaporé. On obtient 32 g de produit huileux dont la composition chimique est la suivante:

| | % mesuré | % théorique |
|---|---|---|
| Soufre | 29,5 | 29,4 |
| Azote | 17,1 | 17,2 |
| Carbone | 44,3 | 44,1 |
| Hydrogène | 9,15 | 9,2 |

## Exemple 2

Dans un ballon tricol d'un litre équipé d'un agitateur mécanique, d'un thermomètre et d'une ampoule à brome, on introduit 600 g d'anhydride polyisobuténysuccinique obtenu à partir d'anhydride maléique et de polyisobutène dont le poids moléculaire moyen est de 900. L'indice d'acide de cet anhydride alcénylsuccinique est de 70 mg de KOH/g. Le produit est chauffé à 155°C. On introduit alors en 30 mn 120 g de tris(amino-5 thia-3 pentyl) amine. Après addition de l'amine, le mélange est placé sous vide (20 mm Hg) à 160°C pendant 2 heures. On obtient un produit limpide composé essentiellement d'un monosuccinimide dérivé de l'amine introduite. La composition chimique de ce produit est la suivante:

| | % mesuré | % théorique |
|---|---|---|
| Soufre | 4,8 | 4,9 |
| Azote | 2,7 | 2,8 |

Exemple 3

Les produits de l'invention obtenus selon les exemples ci-dessus ont été testés sur le plan de leurs propriétés dispersantes dans les lubrifiants. L'étude du pouvoir dispersif a été conduite selon la méthode à la tache décrite dans le tome 1 de l'ouvrage de A. Schilling "Les huiles pour moteurs et le graissage des moteurs" édition 1962 pages 89—90. La méthode est réalisée à partir de 20 g d'huile SAE 30 auxquels sont ajoutés 5 g de Sludge provenant d'un moteur Petter $AV_1$ et contenant environ 2 % de matières charbonneuses.

L'huile SAE 30 a été préalablement additivée à l'aide de la formulation suivante (les quantités des différents additifs sont données pour 1 kg d'huile):

— 40 g de dispersant obtenu aux exemples 1 et 2
— 30 mmoles d'alkylbenzène sulfonate de calcium
— 30 mmoles d'alkylphénate de calcium suralcalinisé
— 15 mmoles de dihexyldithiophosphate de zinc.

Le mélange huile additivée-sludge est séparé en fractions qui sont agitées et soumises aux 5 traitements thermiques suivants:

— une fraction soumise à un chauffage à 50°C pendant 10 mn
— une fraction soumise à un chauffage à 200°C pendant 10 mn
— une fraction soumise à un chauffage à 250°C pendant 10 mn
— une fraction soumise à un chauffage à 50°C pendant 10 mn en présence de 1% d'eau
— une fraction soumise à un chauffage à 200°C pendant 10 mn en présence de 1% d'eau.

Une goutte de chaque mélange obtenu après traitement thermique est déposée sur un papier filtre.

La cotation est effectuée au bout de 48 heures. Pour chaque tache on calcule le pourcentage de produit dispersé par rapport à la tache d'huile, en faisant le rapport des diamètres respectifs de la tache d'huile et du produit dispersé. Plus le pourcentage de produit dispersé est élevé, meilleure est la dispersion vis-à-vis du sludge. Les cotations figurent au tableau final.

Exemple 4

Les propriétés antirouille des produits des exemples 1 et 2 sont testées dans l'huile SAE 30 additivée à l'aide de la formulation de l'exemple précédent à savoir, pour 1 kg d'huile:

— 40 g de produits des exemples 1 et 2
— 30 mmoles d'alkylbenzènesulfonate de calcium
— 30 mmoles d'alkylphénate de calcium suralcalinisé
— 15 mmoles de dihexyldiethiophosphate de zinc.

Le principe du test consiste à ajouter à l'huile étudiée les produits susceptibles de se trouver dans les gaz de blow-by et jouant le rôle dans la formation de la rouille sur l'ensemble poussoir et tige de soupape, et à immerger pendant un certain temps dans le mélange ainsi formé une pièce faisant partie de l'ensemble précédent. La rouille formée est cotée visuellement.

Le test est réalisé par:

— introduction de 700 g d'huile dans un ballon et chauffuage à 50°C sous agitation.

— addition successive, lorsque la température est stabilisée, de 20 cm3 d'une solution aqueuse de formol à 30%, 4,5 cm3 de méthanol, 5 cm3 d'un mélange 50/50 de dichloréthane et dibromoéthane et 8,5 cm3 d'une solution aqueuse d'acide nitrique à 78,5 %,

— immersion pendant 19 h d'une pièce de l'ensemble poussoir et tige de soupape.

Lorsqu'il n'y a pas d'attaque, on attribue au produit la note 20; lorsque l'attaque est très importante, on attribue la note 0. Les résultats des cotations figurent au tableau final.

Exemple 5

Les propriétés antimousse des produits des exemples 1 et 2 sont mesurées selon la norme ASTM D 892 63. Dans l'huile SAE 30 additivée à l'aide de la formulation indiquée aux exemples 3 et 4.

Les résultats des cotations figurent au tableau final.

A titre comparatif on donne dans le tableau final le résultat des tests des propriétés dispersantes, antirouille et antimousse, réalisés dans les conditions décrites aux exemples 3 à 5 sur des succinimides antérieurs, à savoir:

I — Le tris(polyisobuténylsuccinimide) dérivé de la tris (amino-6 oxa-3 hexyl)amine et d'un PIBSA d'indice d'acide 74 obtenu par condensation de l'anhydride maléique et d'un polyisobutène de masse moléculaire voisine de 1000.

II — Le bis(polyisobuténylsuccinimide) dérivé de la triéthylénetriamine et d'un PIBSA d'indice d'acide 74 obtenu par condensation de l'anhydride maléique et d'un polyisobutène de masse moléculaire voisine de 1000.

| Produits | Performances | | |
|---|---|---|---|
| | dispersion | anti-rouille | anti-mousse |
| Celui de l'exemp. 1 | 450 | 18 | 10–05 |
| Celui de l'ex. 2 | 470 | 19 | 10–05 |
| I comparatif | 390 | 12 | 20–50 |
| II comparatif | 320 | 9 | 580–450 |

## Revendications

1. Nouvelles compositions à base d'alcénylsuccinimides, caractérisées en ce qu'elles comprennent au moins un alcényl succinimide de formule:

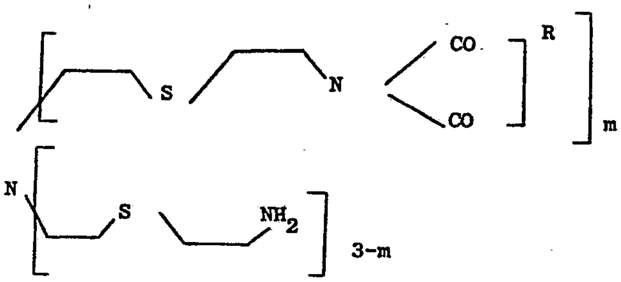

où R représente un groupe alcényl contenant de 20 à 200 atomes de carbone; m est un nombre entier compris entre 1 et 3.

2. Nouvelles compositions selon la revendication 1, caractérisées en ce que le radical R est un groupe polyisobutényle.

3. Procédé de préparation des compositions faisant l'objet de la revendication 1) caractérisé en ce que l'on fait réagir un anhydride alcényl-succinique dans lequel le groupe alcényle renferme de 20 à 200 atomes de carbone, avec de la tris(amino-5 thia-3 pentyl)amine, à une température comprise entre 120 et 230°C, selon un rapport nombre d'équivalents amine/nombre d'équivalents acide compris entre 0,4 et 0,6.

4. Procédé selon la revendication 3) caractérisé en ce que la réaction est réalisée à une température comprise entre 140 et 180°C.

5. Procédé selon la revendication 3) caractérisé en ce que le groupe alcényle est un groupe polyisobutényle.

6. Application des nouvelles compositions faisant l'objet de la revendication 1 ou de la revendication 2 comme additifs pour huiles lubrifiantes.

## Claims

1. New compositions based on alkenyl succinimides, characterized in that they comprise at least one alkenyl succinimide of the formula:

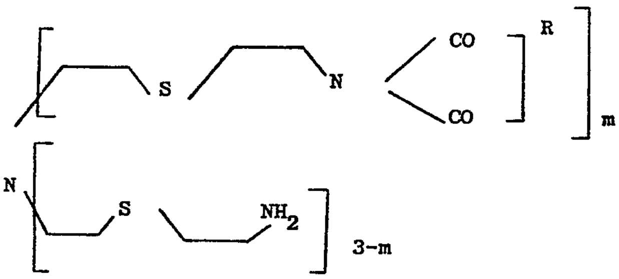

in which R represents an alkenyl-group containing from about 20 to 200 carbon atoms and m is a whole number from 1 to 3.

2. New compositions according to claim 1, characterized in that the radical R is a polyisobutenyl group.

3. A process for the preparation of the new compositions according to claim 1, characterized in that an alkenyl succinic acid anhydride in which the alkenyl group contains from about 20 to 200 carbon atoms, is reacted with tris(5-amino-3-thia-pentyl)amine, in a ratio of number of equivalents of amine to number of equivalents of acid of between about 0.4 and 0.6, at a temperature between about 120° and 230°C.

4. A process according to claim 3, wherein the temperature is between about 140° and 180°C.

5. A process according to claim 3, wherein the alkenyl group is a polyisobutenyl group.

6. Application of the novel compositions according to claim 1 or to claim 2 as lubricating oil additives.

## Patentansprüche

1. Neue Mischungen auf der Basis von Alkenylsuccinimiden, dadurch gekennzeichnet, dass sie mindestens ein Alkenylsuccinimid der Formel

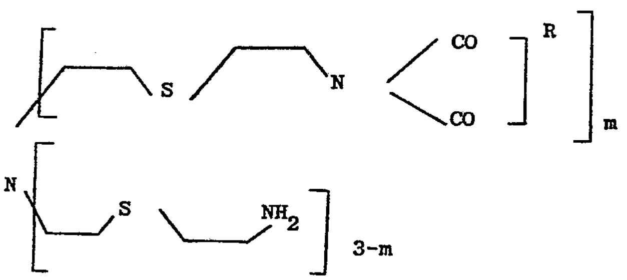

in welcher R eine Alkenylgruppe mit 20 bis 200 Kohlenstoffatomen bedeutet und m für eine ganze Zahl zwischen 1 und 3 steht, enthalten.

2. Neue Mischungen nach Anspruch 1, dadurch gekennzeichnet, dass die Gruppe R eine Polyisobutenylgruppe darstellt.

3. Varfahren zur Herstellung der Mischungen nach Anspruch 1, dadurch gekennzeichnet, dass man ein Alkenyl-bernsteinsäureanhydrid, in welchem die Alkenylgruppe 20 bis 200 Kohlenstoffatome enthält, mit tris-(5-Amino-3-thia-pentyl)-amin, wobei das Verhältnis der Anzahl der Aminäquivalente/Säureäquivalente zwischen 0,4 und 0,6 beträgt, bei einer Temperatur zwischen 120 und 230°C umsetzt.

4. Verfahren nach Anspruch 3, dadurch gekennzeichnet, dass man die Umsetzung bei einer Temperatur zwischen 140 und 180°C durchführt.

5. Verfahren nach Anspruch 3, dadurch gekennzeichnet, dass die Alkenylgruppe eine Polyisobutenylgruppe darstellt.

6. Verwendung der Mischungen gemäss Anspruch 1 oder 2 als Zusatzstoffe für Schmieröle.